# EUROPEAN PATENT APPLICATION

(11) **EP 1 466 633 A1**
(43) Date of publication of application: **13.10.2004**
(21) Application number: 04252019.7
(22) Date of filing: 02.04.2004
(51) Int. Cl.: A61L 27/56, A61L 27/58, A61L 27/44, A61L 27/38

(54) **Composite scaffolds seeded with mammalian cells**

(30) Priority: 02.04.2003 US 405693
(71) Applicant: Lifescan, Inc., Milpitas, California 95035-6312 (US)
(72) Inventor: Rezania, Alireza, Hillsborough New Jersey 08448 (US); Zimmerman, Mark, East Brunswick New Jersey 08816 (US)
(74) Representative: Mercer, Christopher Paul

(57) **Abstract**

Implantable, biocompatible scaffolds containing a biocompatible, porous, polymeric matrix, a biocompatible, porous, fibrous mat encapsulated by and disposed within said polymeric matrix, and a plurality of mammalian cells seeded within said tissue scaffold. The invention also is directed to methods of treating disease or structural defects in a mammal utilizing the scaffolds of the invention.

## Description

### FIELD OF THE INVENTION

The present invention relates to composite tissue scaffolds seeded with mammalian cells for treating a disease or structural defects in soft or hard tissues.

### BACKGROUND OF THE INVENTION

There is a clinical need to treat three classes of diseases that afflict many individuals. The first class of disease relates to diseases/damaged musculoskeletal tissues, such as cartilage, bone, meniscus or muscle. In general, the clinical approaches to repair damaged or diseased musculoskeletal tissue, such as bone, cartilage or muscle, do not substantially restore the original function of the tissue. Prosthetic joints/devices often have been used to treat such defects with mixed outcomes attributed to loosening, limited durability and loss of functional tissue surrounding the defect.

The second class of diseases relates to the loss of organ function, such as diabetes mellitus (DM). DM results from destruction of beta cells in the pancreas or from insensitivity of muscle or adipose tissues to the hormone insulin. The current treatments of DM remain inadequate in averting major health complications, such as blindness, kidney failure and ulcers.

The third class of disease relates to injured or damaged central nervous system (CNS). Injury to spinal cord can lead to destruction of the white and gray matter in addition to blood vessels. Trauma or degenerative processes commonly cause spinal cord injuries. The CNS, unlike many other tissues, has a limited capacity for self-repair because mature neurons lack the ability to regenerate. Previous attempts at regenerating axons in the CNS have included: transplantation of antibodies that block inhibitory proteins; transplantation of glial, macrophage and stem cells; using steroid drugs such as methylpredisolone to reduce the swelling following a CNS injury; and using a support structure in combination with cells or bioactive signals to trigger neuronal regeneration. These approaches have resulted in inadequate repair of the CNS following trauma or disease. Thus, there remains a strong need for alternative approaches for tissue repair/regeneration that avoid the common problems associated with current clinical approaches.

The recent emergence of tissue engineering may offer alternative approaches to repair and regenerate damaged/diseased tissue. Tissue engineering strategies have explored the use of biomaterials in combination with cells and/or growth factors to develop biological substitutes that ultimately can restore or improve tissue function. Scaffold materials have been extensively studied as tissue templates, conduits, barriers and reservoirs useful for tissue repair. In particular, synthetic and natural materials in the form of foams, sponges, gels, hydrogels, textiles and nonwovens have been used in vitro and in vivo to reconstruct/regenerate biological tissue, as well as to deliver chemotactic agents for inducing tissue growth.

Regardless of the composition of the scaffold and the targeted tissue, the scaffold must possess some fundamental characteristics. The scaffold must be biocompatible, possess sufficient mechanical properties to resist loads experienced at the time of surgery; be pliable, be highly porous to allow cell invasion or growth, allow for increased retention of cells in the scaffold; be easily sterilized; be able to be remodeled by invading tissue, and be degradable as the new tissue is being formed. The scaffold may be fixed to the surrounding tissue via mechanical means, fixation devices, sutures or adhesives. So far, conventional materials used in tissue scaffolds, alone or in combination, have proven ineffective to retain seeded cells following implantation.

Accordingly, there is a need for a cell-seeded scaffold that can resolve the limitations of conventional materials.

### SUMMARY OF THE INVENTION

The present invention is directed to implantable, biocompatible scaffolds containing a biocompatible, porous, polymeric matrix, a biocompatible, porous, fibrous mat encapsulated by and disposed within said polymeric matrix; and a plurality of mammalian cells seeded within said tissue scaffold prior to implantation of the scaffold into a defect site or an ectopic site of a mammal. The invention also is directed to methods of treating disease in a mammal utilizing the scaffolds of the invention. The fibrous mat is preferably a nonwoven mat. The porous, biocompatible matrix encapsulating the fibrous mat is preferably a porous, polymeric foam, preferably formed using a lyophilization process.

The present invention allows for enhanced retention of mammalian cells and increased production of the desired extracellular matrix (ECM) within the composite scaffold.

In addition, the cell-seeded composite scaffold can act as a vehicle to deliver cell-secreted biological factors. Such biological factors may direct up-regulation or down-regulation of other growth factors, proteins, cytokines or proliferation of other cell types. A number of cells may be seeded on such a composite scaffold before or after implantation into a defect site or site of diseased tissue.

### BRIEF DESCRIPTION OF THE FIGURES

Figure 1 is a scanning electron micrograph of a portion of a composite scaffold containing a 60/40 PGA/PCL foam encapsulating a 90/10 PGA/PLA nonwoven mat.
Figure 2 is a H&E section of a tissue scaffold of the present invention seeded with mice Sertoli cells.
Figure 3 is a scanning electron micrograph of a portion of a composite scaffold described in Example 12.
Figure 4 is a scanning electron micrograph of a portion of a composite scaffold described in Example 13.
Figure 5 is a scanning electron micrograph of a portion of a composite scaffold described in Example 14.
Figure 6 is fluorescence image of the composite scaffolds seeded with islets described in Example 15.

### DETAILED DESCRIPTION OF THE INVENTION

The present invention is directed to biocompatible composite tissue scaffolds comprising a porous, biocompatible, fibrous mat encapsulated by and disposed within a porous, biocompatible, polymeric matrix. Mammalian cells are administered, i.e. seeded, into the composite scaffold, preferably prior to implantation of the composite scaffold into a defect site or an ectopic site of a mammal.

The present cell-seeded composite scaffold provides an environment whereby administered, i.e. seeded, cells can attach to both fibers of the porous, fibrous mat and to the pore walls of the porous, polymeric matrix encapsulating the fibrous mat. This unique design, combining both the fibrous mat and the porous polymeric matrix, encourages enhanced retention of administered cells within the scaffold, as compared to the use of a porous, fibrous mat or a porous, polymeric matrix alone.

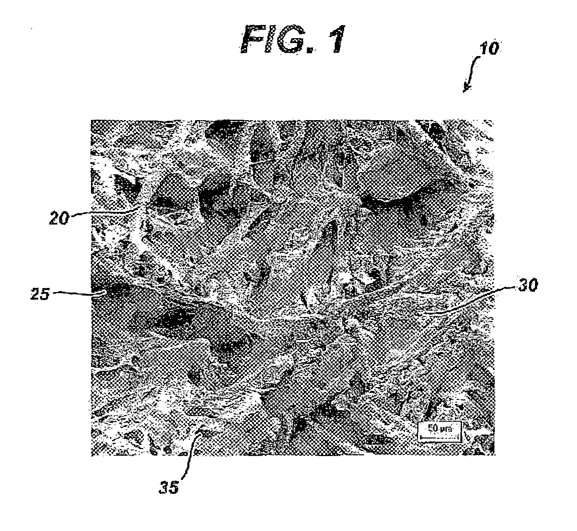

An embodiment of the porous composite scaffold of the present invention is shown in Figure 1. The figure shows composite scaffold 10 comprising a mat of fibers 20 disposed within and encapsulated by porous polymeric matrix 30. Scaffold 10 comprises both macropores 25 and micropores 35. Micropores, as used herein, includes pores having an average diameter of less than about 50 microns. Macropores, as used herein, includes pores having an average diameter of greater than about 50 microns.

After preparation of scaffold 10, mammalian cells are administered, or seeded, within the scaffold prior to, or at the time of, implantation. The mammalian cells may be isolated from vascular or avascular tissues, depending on the anticipated application or the disease being treated. The cells may be cultured under standard conditions known to those skilled in the art in order to increase the number of cells or induce differentiation to the desired phenotype prior to seeding into the scaffold. Alternatively, the isolated mammalian cells may be injected directly into scaffold 10 and then cultured in vitro under conditions promoting proliferation and deposition of the appropriate biological matrix prior to implantation. One skilled in the art, having the benefit of this disclosure, will readily recognize such conditions. In the preferred embodiment, the isolated cells are injected directly into scaffold 10 with no further in vitro culturing prior to in vivo implantation.

The scaffolds of the present invention may be non-biodegradable, i.e. not able to be readily degraded in the body, whereby the degraded components may be absorbed into or passed out of the body, wherein either fibers 20 of said fibrous mat and/or porous, polymeric matrix 30 may comprise non-biodegradable materials. In other embodiments, the scaffolds of the present invention may be biodegradable, i,.e. capable of being readily degraded by the body, wherein the biodegraded components are absorbed into or passed from the body, wherein both the fibrous mat and the polymeric matrix comprise biodegradable materials.

The fibrous mat may comprise non-biodegradable fibers of biocompatible metals, including but not limited to stainless steel, cobalt chrome, titanium and titanium alloys; or bio-inert ceramics, including but not limited to alumina, zirconia and calcium sulfate; or biodegradable glasses or ceramics comprising calcium phosphates; or biodegradable autograft, allograft or xenograft bone tissue.

The porous, polymeric matrix or the fibrous mat may comprise non-biodegradable polymers, including but not limited to polyethylene, polyvinyl alcohol (PVA), polymethylmethacrylte (PMMA), silicone, polyethylene oxide (PEO), polyethylene glycol (PEG), and polyurethanes.

The polymeric matrix may comprise biodegradable biopolymers. As used herein, the term "biopolymer" is understood to encompass naturally occurring polymers, as well as synthetic modifications or derivatives thereof. Such biopolymers include, without limitation, hyaluronic acid, collagen, recombinant collagen, cellulose, elastin, alginates, chondroitin sulfate, chitosan, chitin, keratin, silk, small intestine submucosa (SIS), and blends thereof. These biopolymers can be further modified to enhance their mechanical or degradation properties by introducing cross-linking agents or changing the hydrophobicity of the side residues.

In a preferred embodiment, fibers 20 and porous matrix 30 preferably comprise biodegradable polymers. This will result in a composite scaffold implant device that is fully degradable by the body.

In such biodegradable scaffolds, a variety of biodegradable polymers may be used to make both the fibrous mat and the porous, polymeric matrix which comprise the composite scaffold implant devices according to the present invention and which are seeded with mammalian cells. Examples of suitable biocompatible, biodegradable polymers include polymers selected from the group consisting of aliphatic polyesters, polyalkylene oxalates, polyamides, polycarbonates, polyorthoesters, polyoxaesters, polyamidoesters, polyanhydrides and polyphosphazenes.

Currently, aliphatic polyesters are among the preferred biodegradable polymers for use in making the composite scaffold according to the present invention. Aliphatic polyesters can be homopolymers or copolymers (random, block, segmented, tapered blocks, graft, triblock, etc.) having a linear, branched or star structure. Suitable monomers for making aliphatic homopolymers and copolymers may be selected from the group consisting of, but are not limited to, lactic acid, lactide (including L-, D-, meso and L,D mixtures), glycolic acid, glycolide, ε-caprolactone, p-dioxanone, trimethylene carbonate, δ-valerolactone, β-butyrolactone, ε-decalactone, 2,5-diketomorpholine, pivalolactone, α,α-diethylpropiolactone, ethylene carbonate, ethylene oxalate, 3-methyl-1,4-dioxane-2,5-dione, 3,3-diethyl-1,4-dioxan-2,5-dione, γ-butyrolactone, 1,4-dioxppan-2-one, 1,5-dioxepan-2-one, 6,6-dimethyl-dioxepan-2-one and 6,8-dioxabicycloctane-7-one.

Elastomeric copolymers also are particularly useful in the present invention. Suitable elastomeric polymers include those with an inherent viscosity in the range of about 1.2 dL/g to about 4 dL/g, more preferably about 1.2 dL/g to about 2 dL/g and most preferably about 1.4 dL/g to about 2 dL/g, as determined at 25°C in a 0.1 gram per deciliter (g/dL) solution of polymer in hexafluoroisopropanol (HFIP). Further, suitable elastomers exhibit a high percent elongation and a low modulus, while possessing good tensile strength and good recovery characteristics. In the preferred embodiments of this invention, the elastomer from which the composite scaffold is formed exhibits a percent elongation greater than about 200 percent and preferably greater than about 500 percent. In addition to these elongation and modulus properties, suitable elastomers also should have a tensile strength greater than about 500 psi, preferably greater than about 1,000 psi, and a tear strength of greater than about 50 lbe/inch, preferably greater than about 80 lbs/inch.

Exemplary biodegradable, biocompatible elastomers include, but are not limited to, elastomeric copolymers of ε-caprolactone and glycolide with a mole ratio of ε-caprolactone to glycolide of from about 35/65 to about 65/35, more preferably from 35/65 to 45/55; elastomeric copolymers of ε-caprolactone and lactide where the mole ratio of ε-caprolactone to lactide is from about 35/65 to about 65/35 and more preferably from 35/65 to 45/55; elastomeric copolymers of lactide and glycolide where the mole ratio of lactide to glycolide is from about 95/5 to about 85/15; elastomeric copolymers of p-dioxanone and lactide where the mole ratio of p-dioxanone to lactide is from about 40/60 to about 60/40; elastomeric copolymers of ε-caprolactone and p-dioxanone where the mole ratio of ε-caprolactone to p-dioxanone is from about from 30/70 to about 70/30; elastomeric copolymers of p-dioxanone and trimethylene carbonate where the mole ratio of p-dioxanone to trimethylene carbonate is from about 30/70 to about 70/30; elastomeric copolymers of trimethylene carbonate and glycolide where the mole ratio of trimethylene carbonate to glycolide is from about 30/70 to about 70/30; elastomeric copolymers of trimethylene carbonate and lactide where the mole ratio of trimethylene carbonate to lactide is from about 30/70 to about 70/30, or blends thereof.

The aliphatic polyesters are typically synthesized in a ring-opening polymerization. The monomers generally are polymerized in the presence of an organometallic catalyst and an initiator at elevated temperatures. The organometallic catalyst is preferably tin based, e.g., stannous octoate, and is present in the monomer mixture at a molar ratio of monomer to catalyst ranging from about 10,000/1 to about 100,000/1. The initiator is typically an alkanol (including diols and polyols), a glycol, a hydroxyacid, or an amine, and is present in the monomer mixture at a molar ratio of monomer to initiator ranging from about 100/1 to about 5000/1. The polymerization typically is carried out at a temperature range from about 80°C to about 240°C, preferably from about 100°C to about 220°C, until the desired molecular weight and viscosity are achieved.

One of ordinary skill in the art will appreciate that the selection of a suitable polymer or copolymer for forming the composite scaffolds depends on several factors. The more relevant factors in the selection of the appropriate polymer(s) that is used to form the scaffold include biodegradation (or biodegradation) kinetics; *in vivo* mechanical performance; cell response to the material in terms of cell attachment, proliferation, migration and differentiation; and biocompatibility. Other relevant factors that, to some extent, dictate the *in vitro* and *in vivo* behavior of the polymer include the chemical composition, spatial distribution of the constituents, the molecular weight of the polymer and the degree of crystallinity.

The ability of the material substrate to resorb in a timely fashion in the body environment is critical. But the differences in the degradation time under in vivo conditions also can be the basis for combining two different copolymers. For example, a copolymer of 35/65 ε-caprolactone and glycolide (a relatively fast degrading polymer) is blended with 40/60 ε-caprolactone and lactide copolymer (a relatively slow degrading polymer) to form the composite scaffold. Preferably, the rate of resorption of the composite scaffold by the body approximates the rate of replacement of the scaffold by tissue. That is to say, the rate of resorption of the composite scaffold relative to the rate of replacement of the scaffold by tissue must be such that the structural integrity required of the scaffold is maintained for the required period of time. Thus, devices of the present invention advantageously balance the properties of biodegradability, resorption and structural integrity over time and the ability to facilitate tissue in-growth, each of which is desirable, useful or necessary in tissue regeneration or repair.

In another embodiment, it is desirable to use polymer blends to form structures which transition from one composition to another composition in a gradient-like architecture. Composite scaffolds having this gradient-like architecture are particularly advantageous in tissue engineering applications to repair or regenerate the structure of naturally occurring tissue such as cartilage, e.g. articular, meniscal, septal, tracheal, etc. For example, by blending an elastomeric copolymer of ε-caprolactone and glycolide with an elastic copolymer of ε-caprolactone and lactide (e.g., with a mole ratio of about 5/95) a scaffold may be formed that transitions from a softer spongy material to a stiffer more rigid material in a manner similar to the transition from cartilage to bone. Clearly, one of ordinary skill in the art having the benefit of this disclose will appreciate that other polymer blends may be used for similar gradient effects, or to provide different gradients, e.g. different degradation profiles, stress response profiles or different degrees of elasticity.

The fibers 20 encapsulated by porous matrix 30 of the present invention comprise fibers in a form selected from threads, yarns, nets, laces, felts and nonwovens. Preferably, fibers 20 are in the form of a nonwoven fibrous mat. Known wet-lay or dry-lay fabrication techniques can be used to prepare the fibrous nonwoven mat of the composite scaffold of the present invention.

In another embodiment, the fibers that form the nonwoven fibrous mat of the composite scaffold are made of a biodegradable glass. Bioglass, a silicate containing calcium phosphate glass, or calcium phosphate glass with varying amounts of iron particles added to control degradation time, are examples of materials that could be spun into glass fibers and used in the preparation of the fibrous mat.

Preferably, the fibers that form the nonwoven fibrous mat of the composite scaffold comprise biodegradable polymers, copolymers, or blends thereof. The biodegradable polymers may be selected from the group consisting of polylactic acid (PLA), polyglycolic acid (PGA), ε-polycaprolactone (PCL), polydioxanone (PDO), or copolymers and blends thereof.

Fusing the fibers of the nonwoven fibrous mat of the composite scaffold with another polymer, using a thermal process, can further enhance the structural integrity of the nonwoven mat of the composite scaffold. For example, biodegradable thermoplastic polymer or copolymer, such as ε-polycaprdlactone (PCL) in powder form, may be added to the nonwoven fibrous mat followed by a mild heat treatment that melts the PCL particles, while not affecting the structure of the fibers. This powder possesses a low melting temperature and acts as a binding agent later in the process to increase the tensile strength and shear strength of the nonwoven fibrous mat. The preferred particulate powder size of PCL is in the range of 10-500 micron in diameter, and more preferably 10-150 micron in diameter. Additional binding agents include a biodegradable polymeric binders selected from the group consisting of polylactic acid (PLA), polydioxanone (PDO) and polyglycolic acid (PGA).

Alternatively, the fibers may be fused together by spraying or dip coating the nonwoven mat in a solution of another biodegradable polymer.

In one embodiment, filaments that form the nonwoven mat may be co-extruded to produce a filament with a sheath/core construction. Such filaments comprise a sheath of biodegradable polymer that surrounds one or more cores comprising another biodegradable polymer. Filaments with a fast-degrading sheath surrounding a slower-degrading core may be desirable in instances where extended support is necessary for tissue in-growth.

The porous matrix 30 of the present invention is preferably in the form of a polymeric foam. The polymeric foam of the composite scaffold implant device may be formed by a variety of techniques well known to those having ordinary skill in the art. For example, the polymeric starting materials may be foamed by lyophilization, supercritical solvent foaming, gas injection extrusion, gas injection molding or casting with an extractable material (e.g., salts, sugar or similar suitable materials).

In one embodiment, the polymer foam matrix of the composite scaffold devices of the present invention may be made by a polymer-solvent phase separation technique, such as lyophilization. Generally, however, a polymer solution can be separated into two phases by any one of four techniques: (a) thermally induced gelation/crystallization; (b) non-solvent induced separation of solvent and polymer phases; (c) chemically induced phase separation, and (d) thermally induced spinodal decomposition. The polymer solution is separated in a controlled manner into either two distinct phases or two bicontinuous phases. Subsequent removal of the solvent phase usually leaves a porous matrix having a density less than that of the bulk polymer and pores in the micrometer ranges.

The steps involved in the preparation of these foams include choosing the appropriate solvents for the polymers to be lyophilized and preparing a homogeneous solution of the polymer in the solution. The polymer solution then is subjected to a freezing and a vacuum drying cycle. The freezing step phase-separates the polymer solution and the vacuum drying step removes the solvent by sublimation and/or drying, thus leaving a porous, polymer matrix, or an interconnected, open-cell, porous foam.

Suitable solvents that may be used in the preparation of the foam scaffold component include, but are not limited to, hexafluoroisopropanol (HFIP), cyclic ethers (e.g., tetrahydrofuran (THF) and dimethylene fluoride (DMF)), acetone, methylethyl ketone (MEK), 1,4-dioxane, dimethlycaxbonate, benzene, toluene, N-methyl pyrrolidone, dimethylformamide, chloroform, and mixtures thereof. Among these solvents, a preferred solvent is 1,4-dioxane. A homogeneous solution of the polymer in the solvent is prepared using standard techniques.

One skilled in the art will appreciate that the preferred solvent system will only dissolve the biodegradable polymer of the polymer foam rather than the fibers of the nonwoven mat of the composite scaffold.

The applicable polymer concentration or amount of solvent that may be utilized will vary with each system. Generally, the amount of polymer in the solution can vary from about 0.01% to about 90% by weight and, preferably, will vary from about 0.1% to about 30% by weight, depending on factors such as the solubility of the polymer in a given solvent and the final properties desired in the foam scaffold.

In one embodiment, solids may be added to the polymer-solvent system to modify the composition of the resulting foam surfaces. As the added particles settle out of solution to the bottom surface, regions will be created that will have the composition of the added solids, not the foamed polymeric material.
Alternatively, the added solids may be more concentrated in desired regions (i.e., near the top, sides, or bottom) of the resulting composite scaffold, thus causing compositional changes in all such regions. For example, concentration of solids in selected locations can be accomplished by adding metallic solids to a solution placed in a mold made of a magnetic material (or vice versa).

A variety of types of solids can be added to the polymer-solvent system. Preferably, the solids are of a type that will not react with the polymer or the solvent. Generally, the added solids have an average diameter of less than about 1 mm and preferably will have an average diameter of about 50 to about 500 microns. Preferably the solids are present in an amount such that they will constitute from about 1 to about 50 volume percent of the total volume of the particle and polymer-solvent mixture (wherein the total volume percent equals 100 volume percent).

Exemplary solids include, but are not limited to, particles of demineralized bone, calcium phosphate particles, Bioglass particles or calcium carbonate particles for bone repair, leachable solids for pore creation and particles of biodegradable polymers not soluble in the solvent system that are effective as reinforcing materials or to create pores as they are degraded, non-biodegradable materials, and biologically-derived biodegradable materials.

Suitable leachable solids include nontoxic leachable materials such as salts (e.g., sodium chloride, potassium chloride, calcium chloride, sodium tartrate, sodium citrate, and the like), biocompatible mono and disaccharides (e.g., glucose, fructose, dextrose, maltose, lactose and sucrose), polysaccharides (e.g., starch, alginate, chitosan), water soluble proteins (e.g., gelatin and agarose). The leachable materials can be removed by immersing the foam with the leachable material in a solvent in which the particle is soluble for a sufficient amount of time to allow leaching of substantially all of the particles, but which does not dissolve or detrimentally alter the foam. The preferred extraction solvent is water, most preferably distilled-deionized water. Preferably, the foam will be dried after the leaching process is complete at low temperature and/or vacuum to minimize hydrolysis of the foam unless accelerated degradation of the foam is desired.

Suitable non-biodegradable materials include biocompatible metals such as stainless steel, cobalt chrome, titanium and titanium alloys, and bioinert ceramic particles (e.g., alumina and zirconia particles). Further, the non-biodegradable materials may include polymers such as polyethylene, polyvinylacetate, polymethylmethacrylate, silicone, polyethylene oxide, polyethylene glycol, polyurethanes, and natural biopolymers (e.g., cellulose particles, chitin, keratin, silk, and collagen particles), and fluorinated, polymers and copolymers (e.g., polyvinylidene fluoride).

It is also possible to add solids (e.g., barium sulfate) that will render the composite scaffolds radio opaque. The solids that may be added also include those that will promote tissue regeneration or regrowth, as well as those that act as buffers, reinforcing materials or porosity modifiers.

Suitable biological materials include solid particles of small intestine submucosa (SIS), hyaluronic acid, collagen, alginates, chondroitin sulfate, chitosan, and blends thereof. The solids may contain the entire structure of the biological material or bioactive fragments found within the intact structure.

Mammalian cells are seeded or cultured with the composite scaffolds of the present invention prior to implantation for the targeted tissue. Cells that can be seeded or cultured on the composite scaffolds include, but are not limited to, bone marrow cells, smooth muscle cells, stromal cells, stem cells, mesenchymal stem cells, synovial derived stem cells, embryonic stem cells, umbilical cord blood cells, umbilical Wharton's jelly cells, blood vessel cells, chondrocytes, osteoblasts, precursor cells derived from adipose tissue, bone marrow derived progenitor cells, kidney cells, intestinal cells, islets, beta cells, pancreatic ductal progenitor cells, Sertoli cells, peripheral blood progenitor cells, fibroblasts, glomus cells, keratinocytes, nucleus pulposus cells, annulus fibrosus cells, fibrochondrocytes, stem cells isolated from adult tissue, oval cells, neuronal stem cells, glial cells, macrophages and genetically transformed cells or combination of the above cells. The cells can be seeded on the scaffolds for a short period of time (< 1 day) just prior to implantation, or cultured for longer (> 1 day) period to allow for cell proliferation and extracellular matrix synthesis within the seeded scaffold prior to implantation.

The site of implantation is dependent on the diseased/injured tissue that requires treatment. For example, to treat structural defects in articular cartilage, meniscus, and bone, the cell-seeded composite scaffold will be placed at the defect site to promote repair of the damaged tissue.

Alternatively, for treatment of a disease such as diabetes mellitus, the cell-seeded scaffold may be placed in a clinically convenient site, such as the subcutaneous space, mesentery, or the omentum. In this particular case, the composite scaffold will act as a vehicle to entrap the administered islets in place after *in vivo* transplantation into an ectopic site.

The localization of the administered cells offers a significant advantage in treatment of diabetes mellitis, because the cell-seeded composite scaffold of the present invention forces cell-to-cell contact, while providing a porous structure for transfer of nutrients and vascularization of the graft that is essential for the proper long-term function of islets.

Previous attempts in direct transplantation of islets through injection into the portal circulation has proven inadequate in long-term treatment of diabetes.
Furthermore, numerous methods of encapsulation of allogeneic or xenogeneic islets with biodegradable or nondegradable microspheres have failed to sustain long-term control of blood glucose levels. These failures have been attributed to inadequate vasculature and/or immune rejection of transplanted islets.

Administering xenogeneic or allogeneic islets in combination with allogeneic or xenogeneic Sertoli cells may circumvent the failures. The Sertoli cells may aid in the survival of the islets and prevention of an immune response to the transplanted islets. Xenogeneic, allogeneic, or transformed Sertoli cells can protect themselves in the kidney capsule while immunoprotecting allogeneic or xenogeneic islets. The cell-seeded composite scaffold of the present invention, when co-seeded with Sertoli and islets, and implanted subcutaneously, circumvents the use of the kidney capsule, a clinical site that is difficult to access. The composite scaffold allows for co-localization of the two cell types such that the Sertoli cells can immunoprotect islets that are in close vicinity, while providing an environment that allows for formation of a vascularized bed.

Alternatively, the Sertoli cells may be cultured with the composite scaffold before transplantation into an ectopic site, followed by administration of the islets into the graft site at some later time point. In another embodiment, the islets and Sertoli cells may be injected into the composite scaffold at the same time prior to *in vivo* implantation. In yet another embodiment, the islets or Sertoli cells can be suspended in a biopolymer such as hyaluronic acid, collagen, or alginate, or collagen/laminin materials sold under the tradename MATRIGEL (Collaborative Biomedical Products, Inc., Bedford, MA), or in a synthetic polymer, such as polyethylene glycol, copolymers of polyethylene glycol and polylysine, hydrogels of alkyd polyesters, or a combination thereof, before injection into the scaffold.

In case of central nervous system (CNS) injuries, the composite scaffold can be seeded with a combination of adult neuronal stem cells, embryonic stem cells, glial cells and Sertoli cells. In the preferred embodiment, the composite scaffold can be seeded with Sertoli cells derived from transformed cell lines, xenogeneic or allogeneic sources in combination with neuronal stem cells. The Sertoli cells can be cultured with the composite scaffold for a period before addition of stem cells and subsequent implantation at the site of injury. This approach can circumvent one of the major hurdles of cell therapy for CNS applications, namely the survival of the stem cells following transplantation. A composite scaffold that entraps a large number of Sertoli cells can provide an environment that is more amenable for the survival of stem cells.

In yet another embodiment of the present invention, the cell-seeded composite scaffold may be modified either through physical or chemical means to contain biological or synthetic factors that promote attachment, proliferation, differentiation and extracellular matrix synthesis of targeted cell types. Furthermore, the biological factors may also comprise part of the composite scaffold for controlled release of the factor to elicit a desired biological function. Another embodiment would include delivery of small molecules that affect the up-regulation of endogenous growth factors. Growth factors, extracellular matrix proteins, and biologically relevant peptide fragments that can be used with the matrices of the current invention include, but are not limited to, members of TGF-β family, including TGF-β1, 2, and 3, bone morphogenic proteins (BMP-2, -4, 6, -12, and -13), fibroblast growth factors-1 and -2, platelet-derived growth factor-AA, and -BB, platelet rich plasma, insulin growth factor (IGF-I, IT) growth differentiation factor (GDF-5, -6, -8, -10) vascular endothelial cell-derived growth factor (VEGF), pleiotrophin, endothelin, nicotinamide, glucagon like peptide-I and II, Exendin-4, retinoic acid, parathyroid hormone, tenascin-C, tropoelastin, thrombin-derived peptides, laminin, biological peptides containing cell-and heparin-binding domains of adhesive extracellular matrix proteins such as fibronectin and vitronectin and combinations thereof.

The biological factors may be obtained either through a commercial source or isolated and purified from a tissue.

Furthermore, the polymers and blends comprising the cell-seeded composite scaffold can be used as a therapeutic agent, or drug, release depot. The variety of different therapeutic agents that can be used in conjunction with the present invention is vast. In general, therapeutic agents that may be administered via the compositions of the invention include, without limitation: anti-rejection agents, analgesics, antioxidants, anti-apoptotic agents such as Erythropoietin, anti-inflammatory agents such as anti-tumor necrosis factor α, anti-CD44, anti-CD3, anti-CD154, p38 kinase inhibitor, JAK-STAT inhibitors, anti-CD28, acetoaminophen, Tranilast, cytostatic agents such as Rapamycin, anti-IL2 agents, and combinations thereof.

To form this release depot, the polymer could be mixed with a therapeutic agent prior to forming the composite. Alternatively, a therapeutic agent could be coated onto the polymer, preferably with a pharmaceutically acceptable carrier. Any pharmaceutical carrier can be used that does not dissolve the polymer. The therapeutic agent may be present as a liquid, a finely divided solid, or any other appropriate physical form. Typically, but optionally, the depot will include one or more additives, such as diluents, carriers, excipients, stabilizers or the like.

The amount of therapeutic agent will depend on the particular agent being employed and medical condition being treated. Typically, the amount of agent represents about 0.001 percent to about 70 percent, more typically about 0.001 percent to about 50 percent, most typically about 0.001 percent to about 20 percent by weight of the depot. The quantity and type of polymer incorporated into the therapeutic agent delivery depot will vary depending on the release profile desired and the amount of agent employed.

In another embodiment, the cell-seeded composite scaffold of the present invention can undergo gradual degradation (mainly through hydrolysis) with concomitant release of the dispersed therapeutic agent for a sustained or extended period. This can result in prolonged delivery, e.g. over 1 to 5,000 hours, preferably 2 to 800 hours, of effective amounts. e.g. 0.0001 mg/kg/hour to 10 mg/kg/hour, of the therapeutic agent. This dosage form can be administered as is necessary depending on the subject being treated, the severity of the affliction, the judgment of the prescribing physician, and the like. Following this or similar procedures, those skilled in the art will be able to prepare a variety of formulations.

The structure of the implant must be effective to facilitate tissue ingrowth. A preferred tissue ingrowth-promoting structure is one where the pores of the composite scaffold component are open and of sufficient size to permit cell growth therein. An effective pore size is one in which the pores have an average diameter in the range of from about 50 to about 1,000 microns, more preferably, from about 50 to about 500 microns.

The following examples are illustrative of the principles and practice of the invention, although not limiting the scope of the invention. Numerous additional embodiments within the scope and spirit of the invention will become apparent to those skilled in the art.

In the examples, the polymers and monomers were characterized for chemical composition and purity (NMR, FTIR), thermal analysis (DSC) and molecular weight by conventional analytical techniques.

Inherent viscosities (I.V., dL/g) of the polymers and copolymers were measured using a 50 bore Cannon-Ubbelhode dilution viscometer immersed in a thermostatically controlled water bath at 30°C utilizing chloroform or hexafluoroisopropanol (HFIP) as the solvent at a concentration of 0.1 g/dL.

In these examples certain abbreviations are used. These include PCL to indicate polymerized ε-caprolactone; PGA to indicate polymerized glycolide; PLA to indicate polymerized (L)lactide; and PDO to indicate polymerized p-dioxanone. Additionally, the ratios in front of the copolymer identification indicate the respective mole percentages of each constituent.

### Example 1: Forming a composite scaffold.

A needle-punched nonwoven mat (2 mm in thickness) composed of 90/10 PGA/PLA fibers was made as described below. A copolymer of PGA/PLA (90/10) was melt-extruded into continuous multifilament yarn by conventional methods of making yarn and subsequently oriented in order to increase strength, elongation and energy required to rupture. The yarns comprised filaments of approximately 20 microns in diameter. These yarns were then cut and crimped into uniform 2-inch lengths to form 2-inch staple fiber.

A dry lay needle-punched nonwoven mat was then prepared utilizing the 90/10 PGA/PLA copolymer staple fibers. The staple fibers were opened and carded on standard nonwoven machinery. The resulting mat was in the form of webbed staple fibers. The webbed staple fibers were needle punched to form the dry lay needle-punched, fibrous nonwoven mat.

The mat was scoured with ethyl acetate for 60 minutes, followed by drying under vacuum.

A solution of the polymer to be lyophilized into a foam was then prepared. The polymer used to manufacture the foam component was a 35/65 PCL/PGA copolymer produced by Birmingham Polymers Inc. (Birmingham, AL), with an I.V. of 1.45 dL/g. A 5/95 weight ratio of 35/65 PCL/PGA in 1,4-dioxane solvent was weighed out. The polymer and solvent were placed into a flask, which in turn was put into a water bath and stirred for 5 hours at 70°C to form a solution. The solution then was filtered using an extraction thimble (extra coarse porosity, type ASTM 170-220 (EC)) and stored in a flask.

A laboratory scale lyophilizer, or freeze dryer, (Model Duradry, FTS Kinetics, Stone Ridge, NY), was used to form the composite scaffold. The needle-punched nonwoven mat was placed in a 4-inch by 4-inch aluminum mold. The polymer solution was added into the mold so that the solution covered the nonwoven mat and reached a height of 2 mm in the mold.

The mold assembly then was placed on the shelf of the lyophilizer and the freeze dry sequence begun. The freeze dry sequence used in this example was: 1) -17°C for 60 minutes, 2) -5°C for 60 minutes under vacuum 100 mT, 3) 5°C for 60 minutes under vacuum 20 mT, 4) 20°C for 60 minutes under vacuum 20 mT.

After the cycle was completed, the mold assembly was taken out of the freeze drier and allowed to degas in a vacuum hood for 2 to 3 hours. The composite scaffolds then were stored under nitrogen.

The resulting scaffolds contained the nonwoven fibrous mat encapsulated by and disposed within a polymeric foam matrix. The thickness of the scaffolds was approximately 1.5 mm.

### Example 2: Forming a composite scaffold.

A biodegradable composite scaffold was fabricated following the process of Example 1, except the polymer lyophilized into a foam was a 60/40 PLA/PCL copolymer from Birmingham Polymers Inc., Birmingham, AL, with an I.V. of 1.45 dL/g. The pore size of this composite scaffold was determined using Mercury Porosimetry analysis. The range of pore size was 1-300 µm with a median pore size of 45 µm. Figure 1 is a scanning electron micrograph (SEM) of the cross-section of the composite scaffold. The SEM clearly shows the lyophilized foam scaffold surrounding and encapsulating the nonwoven fibers.

### Example 3: Forming a composite scaffold.

A biodegradable composite scaffold was fabricated following the process of Example 1, except the polymer lyophilized into a foam was a 50:50 blend of 60/40 PLA/PCL and 35/65 PCL/PGA copolymers from Birmingham Polymers Inc., Birmingham, AL, with I.V.s of 1.50 dL/g and 1.45 dL/g, respectively.

### Example 4: Forming a composite scaffold.

A biodegradable composite scaffold was fabricated following the process of Example 1, except the polymer lyophilized into a foam was a 70:30 blend of 60/40 PLA/PCL (Birmingham Polymers Inc., Birmingham, AL) with an I.V. of 1.50 dL/g, and 85/15 PLA/PGA (Purac, Lincolshine, IL) with an I.V. of 1-78 dL/g.

### Example 5: Forming a composite scaffold.

A biodegradable composite scaffold was fabricated following the process of Example 1, except the polymer lyophilized into a foam was a 30:70 blend of 60/40 PLA/PCL (Birmingham Polymers Inc., Birmingham, AL) with an I.V. of 1.50 dL/g, and 85/15 PLA/PGA (Purac, Lincolshine, IL) with an I.V. of 1.78 dL/g.

### Example 6: Forming a composite scaffold.

A biodegradable composite scaffold was fabricated following the process of Example 1, except the polymer lyophilized into a foam was a 50:50 blend of 60/40 PLA/PCL (Birmingham Polymers Inc., Birmingham, AL) with an I.V. of 1.50 dL/g, and 85/15 PLA/PGA (Purac Lincolshine, IL) with an I.V. of 1.78 dL/g.

### Example 7: Forming a composite scaffold.

A biodegradable composite scaffold was fabricated following the process of Example 1, except the dry lay needle-punched nonwoven mat was composed of PDO fibers.

### Example 8: Forming a composite scaffold.

A biodegradable composite scaffold was fabricated following the process of Example 1, except the dry lay needle-punched nonwoven mat was composed of PGA fibers.

### Example 9: Forming a composite scaffold.

A biodegradable composite scaffold was fabricated following the process of Example 4, except the dry lay needle-punched nonwoven mat was composed of PGA fibers.

### Example 10: Forming cell-seeded composite scaffolds.

This example illustrates that the composition of the polymer foam or the dry lay needle-punched nonwoven mat in the composite scaffold affected the *in vitro* response of chondrocytes.

Primary chondrocytes were isolated from bovine shoulders as described by Buschmann, et al., in *J.* *Orthop. Res.,* 10, 745, (1992). Bovine chondrocytes were cultured in Dulbecco's codified eagles medium (DMEM-high glucose) supplemented with 10% fetal calf serum (FCS), 10 mM HEPES, 0.1 mM nonessential amino acids, 20 µg/ml L-proline, 50 µg/ml ascorbic acid, 100 U/ml penicillin, 100 µg/ml streptomycin and 0.25 µg/ml amphotericin B (growth media). Half of the medium was replenished every other day.

Composite scaffolds were prepared as described in Examples 1, 4, 8 and 9. The scaffolds, 5 mm in diameter and 1.5 mm thick, were sterilized for 20 minutes in 70% ethanol followed by five rinses of phosphate-buffered saline (PBS).

Freshly isolated bovine chondrocytes were seeded at a density of 5 x 10⁶ cells/scaffold in 24 well low cluster dishes, by adding a cell suspension (15µl) onto each scaffold. Cells were allowed to attach to the scaffold for three hours before addition of 1.5 ml of medium. Scaffolds were cultured for seven days in cell culture dishes before transferring half of the samples into rotating bio-reactors and culturing the remaining scaffolds under static conditions. The NASA-developed Slow Turning Lateral Vessel (STLV) rotating bio-reactors (Synthecon, Inc., Houston, TX) with simulated microgravity were used,for this study. Each bio-reactor was loaded with four scaffolds containing cells, and the vessel rotation speed was adjusted with the increasing weight of cell-seeded scaffolds. The scaffolds were maintained in a continuous free-fall stage. Scaffolds were incubated for up to 6 weeks in a humidified incubator at 37°C in an atmosphere of 5% CO₂ and 95% air. Half of the medium (~50ml) was replaced every other day for bio-reactor cultures. Static cultures maintained in 6 well dishes were fed with medium (5ml) every other day. Three samples for each time point were evaluated for histological staining. Scaffolds harvested at various time points (1, 7, 21 and 42 days) were fixed in 10% buffered formalin, embedded in paraffin and sectioned using a Zeiss Microtome. Cell distribution within polymer scaffolds was assessed by hematoxylin staining of cross sections of scaffolds 24 hours after cell seeding. Furthermore, sections were also stained for the presence of sulfated proteoglycans using Safranin-O (SO; sulfated GAG's), and immunohistochemically stained for type I and II collagen. Native bovine cartilage and skin were also stained for type I and II collagen to verify the specificity of the immunostains. Collagen type II was used as an indicator of a cartilage-like matrix and type I was used as an indicator of a fibrous-like matrix. Computer images were acquired using a Nikon Microphot-FXA microscope fitted with a Nikon CCD video camera (Nikon, Japan).

Histological sections (100X) of the composite scaffolds formed in Examples 1, 4, 8 and 9 cultured for 6 weeks under bio-reactor conditions were obtained. The composite scaffolds from Example 4, which contained the 90/10 PGA/PLA nonwoven fibers, showed uniform distribution of cells and proteoglycan formation as compared to the composite scaffolds from Example 9, which contained 100% PGA nonwoven fibers. However, histological sections of the two composite scaffolds formed in Examples 1 and 8, cultured for 6 weeks under bio-reactor conditions, showed no significant difference in GAG production and distribution of cells. This shows that the composition of the foam and the nonwoven components of the composite scaffold can affect the distribution of cells and extracellular matrix formation.

In summary, the architecture of the foam scaffold encapsulating a nonwoven fibrous mat supported cell migration and deposition of a sulfated proteoglycan matrix.

### Example 11: Forming cell-seeded composite scaffolds.

This example illustrates that the composition of the polymer foam or the dry lay needle-punched nonwoven mat in the composite scaffold affected the in vitro response of Sertoli cells.

Sertoli cells were harvested from the testes of 9-12 day old male Balb/c mice. Testes were collected in Hank's balanced salt solution (HBSS), chopped into 1-mm pieces, and digested for 10 mins at 37°C with collagenase (2.5 mg/ml; Sigma type V) in HBSS. The digest was rinsed three times with Ca²⁺/Mg²⁺-free HBSS containing 1 mmol/l EDTA and 0.5% bovine serum albumin (BSA), digested for 10 mins at 37°C with trypsin (25 µg/ml Boehringer Mannheim) and Dnase (4 µg/ml, Boehringer Mannheim) in HBSS, followed by four washes in HBSS. The final cell pellet was resuspended in M199 medium (Gibco Life Technologies, Rockville, MD) supplemented with 10% heat-inactivated horse serum, passed through a 500 µm filter and cultured for 2 days in Ultra low cluster dishes (Corning Inc, Corning, NY) to allow aggregation of Sertoli cells.

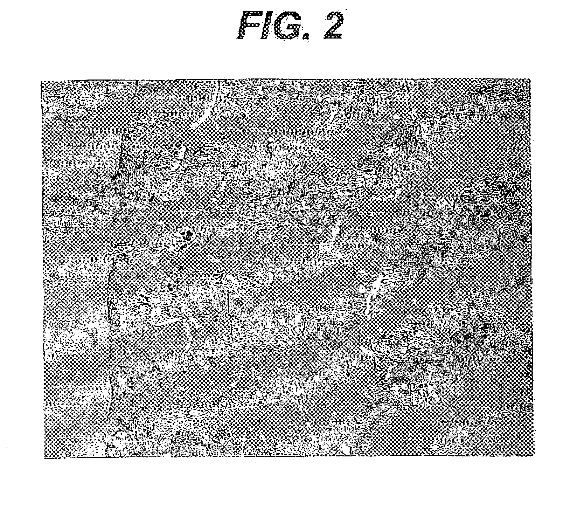

Scaffolds were prepared as in Example 1 and seeded with 1.2 million mice Sertoli cells and cultured for 3 weeks in Ml99 media supplemented with 10% heat-inactivated horse serum and Penicillin and Streptomycin. Following 3 weeks, the devices were fixed in 10% buffered formalin, embedded in paraffin and sectioned using a Zeiss Microtome. Cell distribution within the construct was assessed by hematoxylin&Eosin (H&E) staining. Figure 2 shows an H&E section of the scaffolds with Sertoli cells.

### Example 12 Forming a composite scaffold.

A needle-punched nonwoven mat (2 mm in thickness) composed of 90/10 PGA/PLA fibers was made as described below. A copolymer of PGA/PLA (90/10) was melt-extruded into continuous multifilament yarn by conventional methods of making yarn and subsequently oriented in order to increase strength, elongation and energy required to rupture. The yarns comprised filaments of approximately 20 microns in diameter. These yarns were then cut and crimped into uniform 2-inch lengths to form 2-inch staple fiber.

A dry lay needle-punched nonwoven mat was then prepared utilizing the 90/10 PGA/PLA copolymer staple fibers. The staple fibers were opened and carded on standard nonwoven machinery. The resulting mat was in the form of webbed staple fibers. The webbed staple fibers were needle punched to form the dry lay needle-punched, fibrous nonwoven mat.

The mat was scoured with ethyl acetate for 60 minutes, followed by drying under vacuum.

A 60 ml solution of the polymer to be lyophilized into a foam was then prepared. The polymer used to manufacture the foam component was a 35/65 PCL/PGA copolymer produced by Birmingham Polymers Inc. (Birmingham, AL), with an I.V. of 1.45 dL/g. A 0.25/99.25 weight ratio of 35/65 PCL/PGA in 1,4-dioxane solvent was weighed out. The polymer and solvent were placed into a flask, which in turn was put into a water bath and Stirred for 5 hours at 70°C to form a solution. The solution then was filtered using an extraction thimble (extra coarse porosity, type ASTM 170-220 (EC)) and stored in a flask.

A laboratory scale lyophilizer, or freeze dryer, (Model Duradry, FTS Kinetics, Stone Ridge, NY), was used to form the composite scaffold. Approximately 10ml of the polymer solution was added into a 4-inch by 4-inch aluminum mold to cover uniformly the mold surface. The needle-punched nonwoven mat was immersed into the beaker containing the rest of the solution until fully soaked then position it in the aluminum mold. The remaining polymer solution was poured into the mold so that the solution covered the nonwoven mat and reached a height of 2 mm in the mold.

The mold assembly then was placed on the shelf of the lyophilizer and the freeze dry sequence begun. The freeze dry sequence used in this example was: 1) -17°C for 60 minutes, 2) -5°C for 60 minutes under vacuum 100 mT, 3) 5°C for 60 minutes under vacuum 20 mT, 4) 20°C for 60 minutes under vacuum 20 mT.

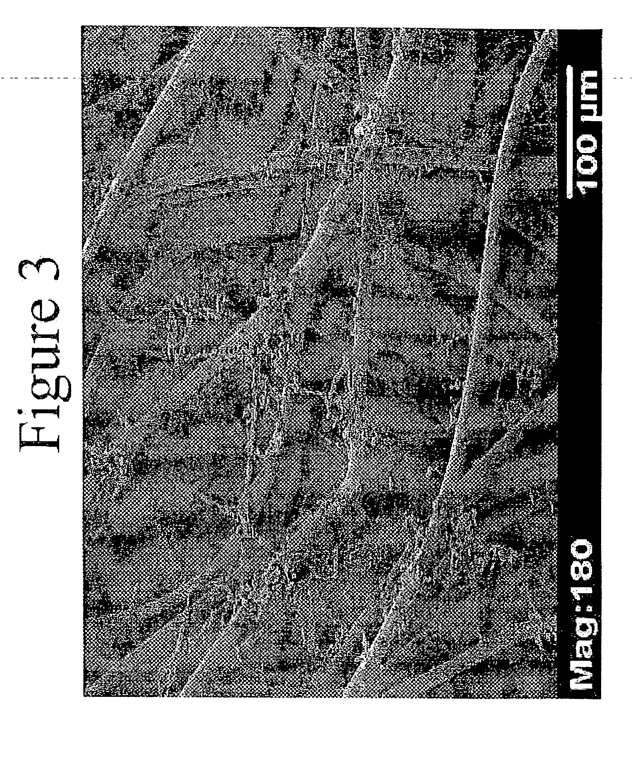

After the cycle was completed, the mold assembly was taken out of the freeze drier and allowed to degas in a vacuum hood for 2 to 3 hours. The composite scaffolds then were stored under nitrogen. Figure 3 is a scanning electron micrograph (SEM) of the cross-section of the composite scaffold. The SEM clearly shows the lyophilized foam scaffold surrounding and encapsulating the nonwoven fibers.

### Example 13

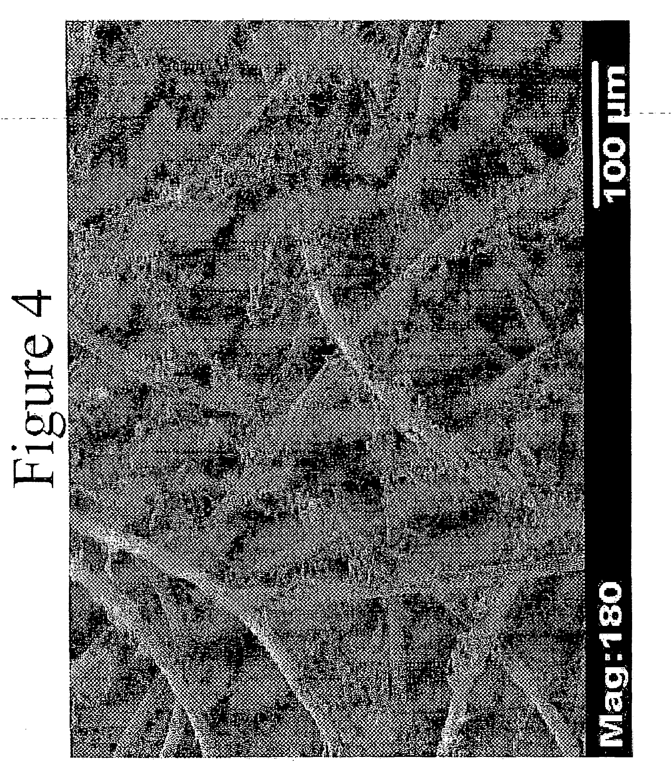

A biodegradable composite scaffold was fabricated following the process of Example 12, except a 0.5/99.50 weight ratio of 35/65 PCL/PGA (Birmingham, AL, with an I.V. of 1.45 dL/g) in 1,4-dioxane solvent was lyophilized to fabricate the foam portion of the composite scaffold. Figure 4 is a scanning electron micrograph (SEM) of the cross-section of the composite scaffold. The SEM clearly shows the lyophilized foam scaffold surrounding and encapsulating the nonwoven fibers.

### Example 14

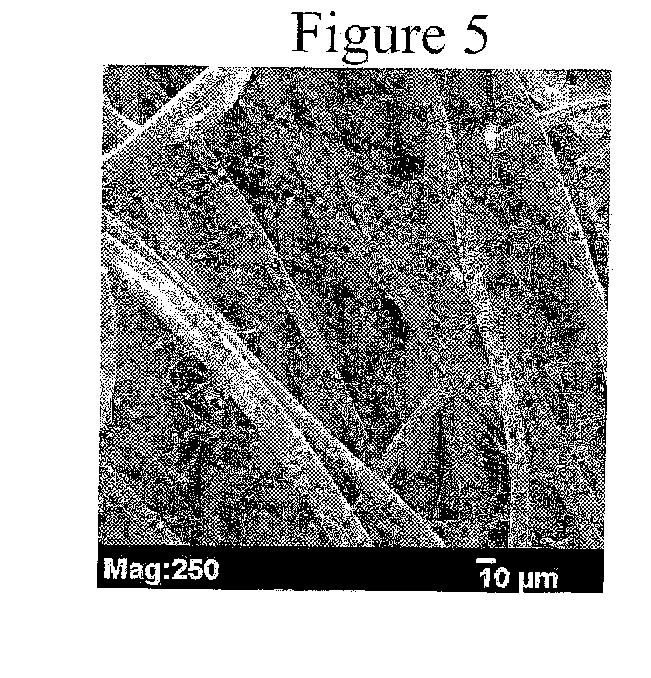

A biodegradable composite scaffold was fabricated following the process of Example 12, except a 1/99 weight ratio of 35/65 PCL/PGA (Birmingham, AL, with an I.V. of 1.45 dL/g) in 1,4-dioxane solvent was lyophilized to fabricate the foam portion of the composite scaffold. Figure 5 is a scanning electron micrograph (SEM) of the cross-section of the composite scaffold. The SEM clearly shows the lyophilized foam scaffold surrounding and encapsulating the nonwoven fibers.

### Example 15 Forming cell-seeded composite scaffolds.

This example illustrates seeding of murine islets onto the composite scaffolds prepared according to Example 12.

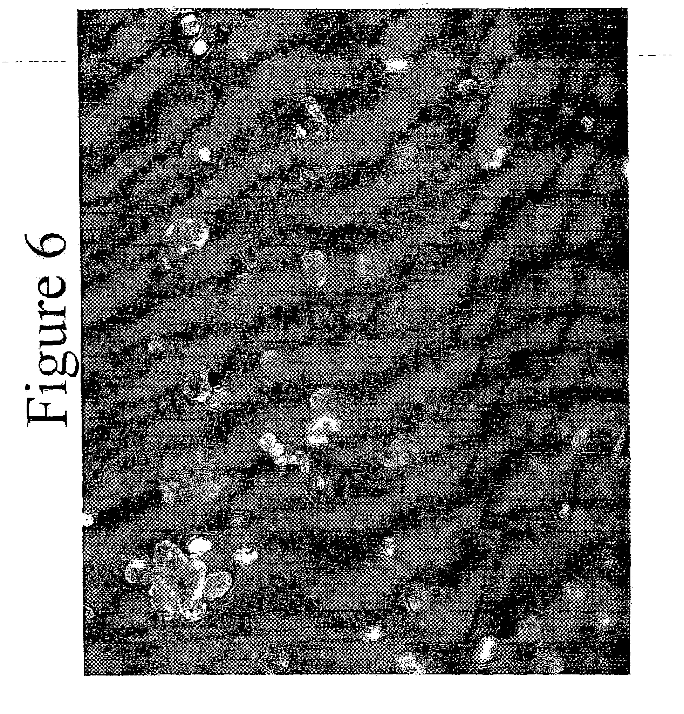

Murine Islets were isolated from Balb/c mice by collagenase digestion of the pancreas and Ficoll density gradient centrifugation followed by hand picking of islets. The composite scaffolds (8 mm in diameter X 2mm in thickness) were placed in a custom made Teflon mold containing multiple wells with a diameter of 7.75 mm. 500 fresh islets were added as a cell suspension (100 microliter volume) over the surface of the scaffolds. The mold containing the cell-seeded constructs was centrifuged for 1 min at 300 RPM. The constructs were removed from the wells and placed in regular cell culture plates and cultured for 1 day in media containing Hams-F10 (Gibco Life Technologies, Rockville, MD) supplemented with bovine serum albumin (0.5%), nicotinamide (10 mM), D-glucose (10 mM), L-glutamine (2 mM), IBMX (3-Isobutyl-1-methylxanthine, 50 mM), and penicillin/Streptomycin. The cell seeded constructs were stained for viability using Live/Dead assay kit (Molecular Probes, Oregon). As shown in Figure 6, the majority of the islets were viable and homogenously distributed through out the scaffold.

## Claims

1. An implantable, biocompatible scaffold, comprising:
a biocompatible, porous, polymeric matrix,
a biocompatible, porous, fibrous mat encapsulated by and disposed within said polymeric matrix; and
a plurality of mammalian cells seeded within said tissue scaffold.

2. The scaffold of claim 1 wherein said scaffold is biodegradable.

3. The scaffold of claim 1 or claim 2 wherein said polymeric matrix comprises a biodegradable polymer and said fibrous mat comprises biodegradable glass fibers, ceramic fibers comprising calcium phosphate or biodegradable polymer fibers.

4. The scaffold of claim 3 wherein said polymeric matrix and said fibrous mat comprise biodegradable polymers.

5. The scaffold of claim 4 wherein said biodegradable polymers are homopolymers or copolymers of aliphatic polyesters, polyalkylene oxalates, polyamides, polycarbonates, polyorthoesters, polyoxaesters, polyamidoesters, polyanhydrides or polyphosphazenes.

6. The scaffold of claim 5 wherein said fibrous mat comprises a 90/10 copolymer of polyglycolide/polylactide.

7. The scaffold of claim 5 wherein said fibrous mat comprises polydioxanone.

8. The scaffold of any one of claims 5 to 7 wherein said polymeric matrix comprises a copolymer of polylactide and polyglycolide in a molar ratio ranging from 95/5 to 85/15 polylactide/polygycolide.

9. The scaffold of any one of claims 5 to 7 wherein said polymeric matrix comprises a copolymer of polycaprolactone and polyglycolide in a molar ratio of from 35/65 to 45/55.

10. The scaffold of any one of claims 5 to 7 wherein said polymeric matrix comprises a copolymer of polylactide and polycaprolactone in a molar ratio of from 35/65 65/35 polylactide/polycaprolactone.

11. The scaffold of any one of claims 1 to 10 wherein said porous, polymeric matrix comprises a foam.

12. The scaffold of any one of claims 1 to 11 wherein said fibrous mat comprises fibers in the form of threads, yams, a net, a laces, a felts or a nonwoven.

13. The scaffold of any one of claims 1 to 12 wherein said mammalian cells are bone marrow cells, smooth muscle cells, stromal cells, stem cells, mesenchymal stem cells, synovial-derived stem cells, embryonic stem cells, blood vessel cells, chondrocytes, osteoblasts, precursor cells derived from adipose tissue, bone marrow derived progenitor cells, kidney cells, intestinal cells, islets, beta cells, Sertoli cells, peripheral blood progenitor cells, fibroblasts, glomus cells, keratinocytes, nucleus pulposus cells, annulus fibrosus cells, fibronchondrocytes, stem cells isolated from adolt tissue, oval cells, neuronal stem cells, glial cells, macrophages or genetically transformed cells.

14. The scaffold of claim 13 wherein said cells are islets or Sertoli cells.

15. The scaffold of claim 13 wherein said cells are adult neuronal stem cells, embryonic stem cells or glial cells.

16. The scaffold of any one of claims 1 to 15 further comprising a biological factor.

17. The scaffold of claim 16 wherein said biological factor is TGF-β1, TGF-β2, TGF-β3, BMP-2, BMP-4, BMP-6, BMP-12, BMP-13, fibroblast growth factor-1, fibroblast growth factor-2, platelet-derived growth factor-AA, platelet-derived growth factor-BB, platelet rich plasma, IGF-I, IGF-II, GDF-5, GDF-6, GDF-8, GDF-10, vascular endothelial cell-derived growth factor, pleiotrophin, endothelin, nicotinamide, glucagon like peptide-I, glucagon like peptide-II, Exendin-4, retinoic acid, parathyroid hormone, tenascin-C, tropoelastin, thrombin-derived peptides, laminin, a biological peptide containing a cell-binding domain or a biological peptide containing a heparin-binding domain.

18. The scaffold of any one of claims 1 to 17 further comprising a therapeutic agent.

19. The scaffold of claim 18 wherein said therapeutic agent is an anti-rejection agent, analgesic, anti-oxidant, anti-apoptotic agent, erythropoietin, anti-inflammatory agent, anti-tumor necrosis factor α, anti-CD44, anti-CD3, anti-CD154, p38 kinase inhibitor, JAK-STAT inhibitor, anti-CD28, acetoaminophen, cytostatic agent, Rapamycin or anti-IL2 agent.

20. The scaffold of any one of claims 1 to 20, for use in therapy, such as the treatment of diabetes mellitus or a structural defect, for instance in articular cartilage, meniscus or bone.
